# EUROPÄISCHE PATENTSCHRIFT

(11) **EP 3 038 535 B1**
(45) Veröffentlichungstag und Bekanntmachung des Hinweises auf die Patenterteilung: **02.08.2017**
(21) Anmeldenummer: 14777532.4
(22) Anmeldetag: 27.08.2014
(51) Int. Cl.: A61B 6/00, G03B 42/04, G06F 19/00

(54) **FACHABLAGE ZUM ABLEGEN VON RÖNTGENBILDPLATTEN UND EIN VERFAHREN ZUR REGISTRIERUNG**
FILING COMPARTMENT SYSTEM FOR FILING X-RAY IMAGE PLATES, AND A RECORDING METHOD
BAC COMPARTIMENTÉ POUR RANGER DES PLAQUES RADIOGRAPHIQUES ET PROCÉDÉ D'ENREGISTREMENT

(30) Priorität: 27.08.2013 DE 102013217041
(43) Veröffentlichungstag der Anmeldung: 06.07.2016
(73) Patentinhaber: Sirona Dental Systems GmbH, 64625 Bensheim (DE)
(72) Erfinder: SCHULZE-GANZLIN, Ulrich, 64653 Lorsch (DE)
(74) Vertreter: Sommer, Peter
(86) Internationale Anmeldenummer: PCT/EP2014/068140
(87) Internationale Veröffentlichungsnummer: WO 2015/028494

(56) Entgegenhaltungen:
- EP-A1- 2 386 904
- WO-A1-2010/109064
- US-A- 5 264 684

## Beschreibung

### Technisches Gebiet

Die Erfindung betrifft eine Fachablage umfassend mehrere Fächer zum Ablegen von Röntgenbildplatten, wie beispielsweise von Speicherfolien oder Röntgenfilmen.

### Stand der Technik

Aus dem Stand der Technik sind mehrere Fachablagen zum Ablegen von Röntgenbildplatten sowie Registrierungsverfahren bekannt. Beispielsweise erwähnt die US-A5,264,684 eine Ablage, in der Röntgenfilme gelagert werden, bevor sie einem Scanner zugeführt werden. Zum Ablegen von Röntgenbildplatten, wie Speicherfolien oder Röntgenfilmen, wird oft eine einfache Fachablage mit mehreren Fächern verwendet. Nach der Belichtung werden die Speicherfolien der Reihe nach in einer solchen Fachablage abgelegt. Anschließend werden die Speicherfolien zum Scannen einzeln herausgenommen und einem Speicherfolienscanner zugeführt. Die belichteten Speicherfolien werden meist manuell, beispielsweise durch eine Nummerierung, gekennzeichnet, um die einzelnen Aufnahmen zu identifizieren und eine Zuordnung zum jeweiligen Patienten nicht zu verlieren und die Speicherfolien nicht zu vertauschen. Im Falle der Filmentwicklung können nach dem Entwickeln die einzelnen Röntgenfilme gescannt werden, um digitale Aufnahmen zu erzeugen. Durch das manuelle Ablegen der Röntgenfilme ist eine Gefahr der Verwechslung wie bei den Speicherfolien gegeben. Ein Nachteil dieses Verfahrens ist, dass durch mangelnde Aufmerksamkeit des Bedienpersonals die Speicherfolien nach der Belichtung falsch gekennzeichnet werden können oder in der falschen Reihenfolge in die Fachablage abgelegt werden können, so dass es zu Verwechslungen hinsichtlich der Reihenfolge kommt. Auch könnte eine einem anderen Patienten zugeordnete Fachablage verwendet werden. Dadurch können Röntgenaufnahmen dem falschen Patienten und der falschen Aufnahmeregion zugeordnet werden, so dass diese Verwechslungen zu Fehldiagnosen und zum Schaden am Patienten führen können. Falls solche Verwechslungen erkannt werden, können diese meist nicht zurückverfolgt werden, so dass eine Wiederholung der Aufnahme erforderlich ist. Neben dem Zeitverlust einer zusätzlichen Aufnahme ist auch die zusätzliche Strahlenbelastung nachteilig.

In der WO 2010/109064 A1 ist ein medizinisches Röntgenbildgebungssystem offenbart, das geeignet ist, Röntgenbildspeicherfolien auszulesen. Röntgenspeicherfolien sind dabei mit elektronisch auslesbaren Identifikationsmarkierungen, den so genannten Tags, versehen, die eine Identifizierung ermöglichen und damit die Gefahr vor Verwechslungen ausschließen.

Ein Nachteil dieses Verfahrens ist, dass die einzelnen Röntgenspeicherfolien, mit solchen Identifizierungsmarkierungen versehen werden müssen. Darüber hinaus müssen diese Identifizierungsmarkierungen unter Verwendung entsprechender Lesegeräte am Röntgengerät und am Speicherfolienscanner ausgelesen werden. Dies ist mit einem erheblichen technischen Aufwand verbunden.

Die Aufgabe der folgenden Erfindung besteht daher darin, eine Fachablage und ein Verfahren zur Registrierung bereitzustellen, die die Gefahr von Verwechslungen verhindern und mit möglichst wenig technischem Aufwand realisiert werden können.

### Darstellung der Erfindung

Die Erfindung betrifft eine Fachablage umfassend mehrere Fächer zum Ablegen von Röntgenbildplatten. Die Fachablageweist ein Registrierungssystem auf, das beim Ablegen einer Röntgenbildplatte in eines der Fächer und/oder beim Entnehmen einer Röntgenbildplatte aus einem der Fächer aktivierbar ist und dadurch ein Aktivierungssignal erzeugt. Zusätzlich ist eine Zeitmessvorrichtung vorhanden, die auf das Aktivierungssignal hin einen Ablegezeitpunkt und/oder einen Entnahmezeitpunkt für die einzelnen Fächer ausgibt, sobald das Registrierungssystem aktiviert wird. Bei Verwendung des Registrierungssystems sind der Ablegezeitpunkt und/oder der Entnahmezeitpunkt sowie eine Fach-Identifikation des jeweiligen Fachs und/oder einer Fachablage-Indikationen der jeweiligen Fachablage mittels eines Datenspeichers registrierbar.

Die Fachablage entspricht in seinen Abmessungen einer herkömmlichen Fachablage zum Ablegen von Röntgenbildplatten, wie von Speicherfolien. Der Unterschied besteht darin, dass die Fachablage das Registrierungssystem aufweist, das das Aktivierungssignal erzeugt.

Die Röntgenbildplatten können unterschiedliche plattenförmige Röntgendetektoren, wie Röntgenfilme, Speicherfolien oder elektronische Röntgenbilddetektoren, sein. Die Zeitmessvorrichtung kann in die Fachablage integriert oder außerhalb der Fachablage angeordnet sein. Falls die Zeitmessvorrichtung außerhalb der Fachablage angeordnet ist, kann das Aktivierungssignal drahtlos oder drahtgebunden an die Zeitmessvorrichtung übermittelt werden. Die Zeitmessvorrichtung gibt den Ablegezeitpunkt und/oder den Entnahmezeitpunkt für jedes Fach aus, wobei diese mit der Fach-Identifikation des Fachs registriert werden. Dabei können beispielsweise mehrere Datensätze in einer Tabelle im Datenspeicher abgelegt werden, die eine eindeutige Zuordnung des Ablegezeitpunkts und/oder des Entnahmezeitpunkts zu dem jeweiligen Fach erlaubt. Die Fach-Identifikation kann beispielsweise eine Identifikationsnummer des Fachs sein.

Ein Vorteil des Fachablage besteht darin, dass die Röntgenbildplatten in einer beliebigen Reihenfolge in die Fächer der Fachablage abgelegt werden können, wobei der Ablegezeitpunkt und somit die Ablegereihenfolge registriert werden. Beim Entnehmen der Röntgenbildplatten aus den Fächern der Fachablage kann dann mittels der registrierten Zuordnung zwischen jedem Fach und dem jeweiligen Ablegezeitpunkt jede der Röntgenbildplatten eindeutig identifiziert werden. Auf diese Weise werden Verwechslungen der Röntgenbildplatten hinsichtlich Patient und/oder Aufnahmeregion aufgrund mangelnder Aufmerksamkeit des Bedienpersonals verhindert.

Vorteilhafterweise kann das Registrierungssystem mindestens einen Sensor für jedes der Fächer aufweisen, wobei die Fachablage eine Auswerteelektronik aufweist, wobei beim Ablegen der Röntgenbildplatte in eines der Fächer und/oder beim Entnehmen der Röntgenbildplatte aus einem der Fächer der jeweilige Sensor aktiviert wird und das Aktivierungssignal erzeugt wird.

Bei dieser Alternative kann jedes Fach einen eigenen Sensor aufweisen, der mit der Auswerteelektronik versehen sein kann. Alternativ kann die Auswerteelektronik beispielsweise ein gemeinsamer µ-Prozessor sein, an dem jeder der Sensoren angeschlossen wird. Auf diese Weise wird der Ablegezeitpunkt für jedes der Fächer der jeweiligen Fach-Identifikation des Fachs zugeordnet. Die Sensoren können beliebig gestaltet sein.

Die Sensoren können auch mit Lichtleitern versehen sein, die Lichtschranken für jedes der Fächer bilden. Vorteilhafterweise können die Sensoren Lichtschranken sein, die eine Lichtquelle und einen optischen Sensor aufweisen. Dadurch wird die Detektion des Ablegezeitpunkts und/oder des Entnahmezeitpunkts verbessert. Die Lichtschranken können beispielsweise am oberen Ende der Fächer angeordnet sein, um eine schnelle Detektion zu ermöglichen. Vorteilhafterweise können die Sensoren Drucksensoren sein, die auf einen bestimmten Druck hin ein Aktivierungssignal abgeben.

Dadurch wird die Detektion des Ablegezeitpunkts und/oder des Entnahmezeitpunkts durch das Auslösen der Drucksensoren ermöglicht. Beim Ablegen einer der Röntgenbildplatten wird der jeweilige Drucksensor belastet und gibt daraufhin das Aktivierungssignal ab.

Vorteilhafterweise können die Sensoren eine Bewegung der Röntgenbildplatte relativ zum jeweiligen Fach beim Ablegen und/oder beim Entnehmen detektieren. Die Sensoren können die Bewegung der Röntgenbildplatte auf der Grundlage eines induktiven, kapazitiven und/oder magnetischen Detektionsverfahrens detektieren.

Dadurch wird mittels der Sensoren eine zuverlässige und genaue Ermittlung des Ablegezeitpunkts und/oder des Entnahmezeitpunkts für jedes der Fächer ermöglicht.

Vorteilhafterweise kann das Registrierungssystem eine Kamera aufweisen, die in kurzen zeitlichen Abständen mehrere Bilder oder Bildsequenzen der Fachablage aufnimmt, wobei mittels einer Analyseeinheit, wie eines Microcomputers oder einer IC-Schaltung, durch computergestützten Vergleich der einzelnen Bilder beim Ablegen und/oder beim Entnehmen der Röntgenbildplatte der Ablegezeitpunkt und/oder der Entnahmezeitpunkt sowie die Fach-Identifikation des jeweiligen Fachs feststellbar sind.

Bei dieser zweiten Alternative ist das Registrierungssystem eine Kamera, die den Zustand der einzelnen Fächer überwacht. Auf diese Weise werden der Ablegezeitpunkt und/oder der Entnahmezeitpunkt zuverlässig ermittelt. Im Unterschied zur ersten Alternative hat diese zweite Alternative den Vorteil, dass die Integration der Sensoren in die einzelnen Fächer entfällt und dadurch der Produktionsaufwand solcher Fachablagen vermindert wird.

Vorteilhafterweise können die Bilder unter Verwendung eines Mustererkennungsverfahrens analysiert werden, um den Belegungsstatus der einzelnen Fächer festzustellen.

Die Auswertung der Bilder kann mittels eines speziellen Algorithmus erfolgen, der die einzelnen Fächer bzw. charakteristische Strukturen der Fachablage oder der abzulegenden Röntgenbildplatten mittels des Mustererkennungsverfahrens identifiziert und den Belegungsstatus der Fächer automatisiert überwacht.

Vorteilhafterweise kann die Fachablage eine Identifikationselektronik aufweisen, die beim Ablegen und/oder beim Entnehmen der Röntgenbildplatte die Fach-Identifikation des jeweiligen Fachs ausgibt.

Dadurch wird die Fach-Identifikation der einzelnen Fächer vereinfacht. Eine Identifikationselektronik ordnet jedem Sensor das jeweilige Fach zu. Bei einer drahtgebundenen Lösung können die einzelnen Sensoren anhand der entsprechenden Leiterbahn identifiziert werden. Bei einer drahtlosen Lösung können die einzelnen Sensoren anhand eines eindeutigen Codes identifiziert werden, der zusammen mit den Registrierungsinformationen übermitteln wird.

Vorteilhafterweise kann die Fachablage an eine Auslesevorrichtung für Röntgenbildplatten unter Verwendung von Befestigungsmitteln anbringbar oder in die Auslesevorrichtung integriert sein.

Dadurch kann ein manueller oder auch ein automatisierter Entnahmevorgang der Röntgenbildplatten aus der Fachablage beschleunigt werden. Bei einem automatisierten Entnahmevorgang sind mechanische Entnahmemittel vorgesehen, die die Röntgenbildplatten automatisch aus der Fachablage entnehmen und der Auslesevorrichtung zuführen.

Vorteilhafterweise kann die Zeitmessvorrichtung in die Fachablage, in eine Bildarchivierungsvorrichtung oder in die Auslesevorrichtung integriert sein.

Das Aktivierungssignal wird dann drahtlos oder drahtgebunden an die Zeitmessvorrichtung übermittelt.

Ebenso können weitere Registrierdaten, wie Ablegezeitpunkt oder Entnahmezeitpunkt, Fachablage-Identifikation, Fach-Identifikation und bestimmte Eigenschaften der jeweiligen Röntgenbildplatte, an einen Datenspeicher übermittelt werden.

Vorteilhafterweise kann der Datenspeicher in die Fachablage, in die Auslesevorrichtung oder in eine Bildarchivierungsvorrichtung integriert sein.

Der Datenspeicher kann ein beliebiges Speichermedium, wie ein Speicherchip, sein.

Vorteilhafterweise können die Sensoren charakteristische Eigenschaften der Röntgenbildplatte detektieren, wobei diese Informationen zusammen mit dem Ablegezeitpunkt und/oder dem Entnahmezeitpunkt mittels des Datenspeichers registrierbar sind.

Diese charakteristischen Eigenschaften können beispielsweise Form, Farbe, optische Merkmale und/oder die Abmessungen der Röntgenbildplatten sein.

Zusätzlich können weitere Informationen über den Patienten und das Aufnahmegebiet der jeweiligen Aufnahme registriert werden.

Die Fachablage kann auch eine Sendeeinheit umfassen, die die Registrierungsinformationen über die charakteristischen Eigenschaften der Röntgenbildplatte, den Ablegezeitpunkt, den Entnahmezeitpunkt, Fachablage-Identifikation und/oder die Fach-Identifikation drahtlos an einen Datenspeicher übermittelt.

Vorteilhafterweise kann die Fachablage eine Sendeeinheit umfassen, die das Aktivierungssignal an die Zeitmessvorrichtung übermittelt.

Die Sendeeinheit kann drahtlos sein und das Aktivierungssignal drahtlos an die Zeitmessvorrichtung übermitteln.

Dadurch kann die Fachablage innerhalb der Reichweite der Sendeeinheit während der Registrierung beliebig bewegt werden.

Die Erfindung betrifft weiterhin ein Verfahren zur Registrierung von Röntgenbildplatten, wobei mehrere Röntgenbildplatten in einer Fachablage abgelegt werden, wobei die Fachablage mehrere Fächer aufweist. Die Fachablage weist ein Registrierungssystem auf, das beim Ablegen einer Röntgenbildplatte in eines der Fächer und/oder beim Entnehmen einer Röntgenbildplatte aus einem der Fächer aktiviert wird und dadurch ein Aktivierungssignal erzeugt wird. Zusätzlich ist eine Zeitmessvorrichtung vorhanden, die auf das Aktivierungssignal hin einen Ablegezeitpunkt und/oder einen Entnahmezeitpunkt für die einzelnen Fächer ausgibt, sobald das Registrierungssystem aktiviert wird. Beim Auslösen des Registrierungssystems werden der Ablegezeitpunkt, der Entnahmezeitpunkt, eine Fach-Identifikation des jeweiligen Fachs und/oder eine Fachablage-Identifikation der jeweiligen Fachablage mittels eines Datenspeichers registriert.

Dieses Verfahren ermöglicht die Registrierung mittels der oben erläuterten Fachablage. Der Vorteil dieses Verfahrens liegt darin, dass Verwechslungen von Röntgenbildplatten bei der Registrierung ausgeschlossen werden.

Ein weiterer Vorteil des Verfahrens liegt darin, dass die Röntgenbildplatten in einer beliebigen Reihenfolge aus der Fachablage herausgenommen und ausgelesen werden können. Eine bestimmte Reihenfolge muss nicht beachtet werden, da die Zuordnung der Aufnahmen automatisch erfolgt.

Die Röntgenbildplatten können Speicherfolien, Röntgenfilme oder elektronische Röntgendetektoren sein. Die Auslesevorrichtung ist dann entsprechend ausgestaltet. Bei Speicherfolien ist die Auslesevorrichtung ein besonderer Scanner. Bei Röntgenfilmen ist die Auslesevorrichtung aus einer Filmentwicklungseinheit und einer Filmscaneinheit aufgebaut, wobei die Röntgenfilme mittels der Filmentwicklungseinheit entwickelt werden und anschließend mittels der digitalen Filmscaneinheit gescannt werden.

Vorteilhafterweise kann das Registrierungssystem jeweils einen Sensor für jedes der Fächer aufweisen, die jeweils mit einer Auswerteelektronik versehen sind, wobei beim Ablegen der Röntgenbildplatte in eines der Fächer und/oder beim Entnehmen der Röntgenbildplatte aus einem der Fächer der jeweilige Sensor aktiviert wird und das Aktivierungssignal erzeugt wird.

Bei dieser ersten Alternative werden der Ablegezeitpunkt und/oder der Entnahmezeitpunkt mittels der in den Fächern angeordneten Sensoren ermittelt.

Vorteilhafterweise kann das Registrierungssystem eine Kamera aufweisen, die zur Überwachung in kurzen zeitlichen Abständen mehrere Bilder der Fachablage aufnimmt. Die Fachablage ist so ausgestaltet, dass durch einen computergestützten Vergleich der einzelnen Bilder beim Ablegen und/oder beim Entnehmen der Röntgenbildplatte der Ablegezeitpunkt, Fachablage-Identifikation und/oder der Entnahmezeitpunkt sowie die Fach-Identifikation des jeweiligen Fachs feststellbar sind.

Bei dieser zweiten Alternative werden der Ablegezeitpunkt und/oder der Entnahmezeitpunkt mittels der Kamera als Registrierungssystem ermittelt.

Vorteilhafterweise können die Bilder unter Verwendung eines Mustererkennungsverfahrens analysiert werden, um den Belegungsstatus der einzelnen Fächer festzustellen.

Dadurch werden die Bilder der Kamera analysiert, wobei die einzelnen Fächer mittels des Mustererkennungsverfahrens erkannt werden und auf diese Weise der Belegungsstatus kontinuierlich überwacht werden kann.

Vorteilhafterweise können die Röntgenbildplatten aus der Fachablage herausgenommen werden und mittels einer Auslesevorrichtung die Röntgenbildplatten ausgelesen werden, wobei digitale Aufnahmen eines Objekts erzeugt werden.

Dadurch werden die digitalen Aufnahmen des Objekts beim Auslesen der Röntgenbildplatten erzeugt. Diese Aufnahmen werden unter Verwendung der registrierten Informationen in der richtigen Reihenfolge angeordnet, so dass Verwechslungen ausgeschlossen werden.

Vorteilhafterweise können unter Verwendung der Zuordnung zwischen dem registrierten Ablegezeitpunkt und der registrierten Fach-Identifikation für jedes der Fächer die erzeugten digitalen Aufnahmen mittels der Bildarchivierungsvorrichtung so umsortiert werden, dass sie nach einer ursprünglichen Ablagereihenfolge angeordnet sind.

Dadurch entsprechen die Aufnahmen nach dem Umsortieren der ursprünglichen Ablagereihenfolge, zum Beispiel entsprechend einer standardisierten Aufnahmeabfolge, so dass die Beurteilung dieser Aufnahmen durch den Benutzer erleichtert wird.

Vorteilhafterweise kann eine Fachablage eine Paarungstaste aufweisen, wobei beim Betätigen der Paarungstaste die jeweilige Fachablage beispielsweise zu Beginn einer Aufnahmeserie einem zu röntgenden Patienten zugeordnet wird. Beim Betätigen der Paarungstaste sendet die Fachablage seine Fachablage-Identifikation an das Registrierungssystem. Dem Anwender werden daraufhin vorzugsweise auf einer Anzeige die jeweilige Fachablage-Identifikation und der zu röntgende Patientenname zur Bestätigung angezeigt. Nach der Bestätigung sind damit Fachablage und Patient gepaart und für die Ablage der Röntgenbildplatten vorbereitet. Dabei kann dem Anwender die Identität der Fachablage und ein Patientenname des zu röntgenden Patienten mittels einer Anzeigeeinheit der Bildarchivierungseinrichtung oder der Auslesevorrichtung angezeigt werden, wobei die Paarung unter Verwendung von Bedienelemente an der Bildarchivierungseinrichtung oder an der Auslesevorrichtung durch den Benutzer manuell bestätigt werden kann.

Die Paarungstaste kann also vor dem ersten Ablegen der Röntgenbildplatten in die jeweilige Fachablage betätigt werden, wobei daraufhin eine Sendeeinheit ein eindeutiges Fachablage-Identifikationssignal an die Bildarchivierungsvorrichtung oder an die Auslesevorrichtung übermittelt.

Alternativ dazu kann auch auf die Paarungstaste zur Paarung verzichtet werden. Dies könnte mit einem Näherungssensor erfolgen, wobei die Fachablage für einen kurzen Moment an eine bestimmte Position gebracht wird. Technisch kann dies mit einem RFID-Reader (radio-frequency identification) erfolgen, der über USB an einen PC angeschlossen ist und den RFID-Chip der Fachablage ausliest. Der Paarungsvorgang wird vervollständigt, wenn das Registrierungssystem, beispielsweise die PC-Software, die identifizierte Fachablage mit dem zu röntgenden Patienten verknüpft und das Paarungsergebnis dem Anwender anzeigt und ggf. vom Anwender bestätigen lässt. Dabei kann dem Anwender die Identität der Fachablage und ein Patientenname des zu röntgenden Patienten mittels einer Anzeigeeinheit der Bildarchivierungseinrichtung oder der Auslesevorrichtung angezeigt werden, wobei die Paarung unter Verwendung von Bedienelemente an der Bildarchivierungseinrichtung oder an der Auslesevorrichtung durch den Benutzer manuell bestätigt werden kann.

Die Sendeeinheit kann also in jeder der Fachablagen einen RFID-Chip aufweisen. Die jeweilige Fachablage wird dann vor dem Auslesen der Röntgenbildplatten in den Empfangsradius eines RFID-Readers gebracht, wobei daraufhin der jeweilige RFID-Chip ein eindeutiges Identifikationssignal an den RFID- Reader übermittelt.

Dadurch werden die einzelnen Fachablagen eindeutig identifiziert, so dass eine Verwechslung vermieden wird. Beispielsweise können einzelne Aufnahmeserien von mehreren Patienten durchgeführt werden und die beleuchteten Röntgenbildplatten der Aufnahmeserien in unterschiedlichen Fachablagen abgelegt werden.

Die Identifizierung der Fachablage kann auch manuell erfolgen, indem der Benutzer die jeweils auszulesende Fachablage anhand deren Farbe, Kennzeichnung oder anderer Merkmale identifiziert und in einem Auswahlmenü an der Bildarchivierungsvorrichtung auswählt.

Die identifizierte Fachablage kann mittels der Bildarchivierungsvorrichtung einem Patientennamen zugewiesen werden. Zur weiteren Kontrolle kann der Patientenname an einem Display der jeweiligen Fachablage und/oder an einem Display an der Auslesevorrichtung angezeigt werden.

Dadurch wird die Gefahr einer Verwechslung der Röntgenbildplatten zusätzlich minimiert.

Vorteilhafterweise kann die Empfangseinheit für die Registrierdaten in die Bildarchivierungsvorrichtung oder in die Auslesevorrichtung integriert sein.

Dadurch kann die Bildarchivierungsvorrichtung direkt oder indirekt über die Auslesevorrichtung das Identifizierungssignal der auszulesenden Fachablage empfangen.

### Kurzbeschreibung der Zeichnungen

Die Erfindung wird anhand der Zeichnungen erläutert. Es zeigt, die
- Fig. 1: eine Fachablage mit mehreren Sensoren, die
- Fig. 2: eine Fachablage mit einer Kamera, die
- Fig. 3: eine Skizze zur Verdeutlichung des Registrierungsverfahrens mit einer Fachablage, die
- Fig. 4: eine Skizze zur Verdeutlichung des Registrierungsverfahrens mit mehreren Fachablagen.

### Ausführungsbeispiel

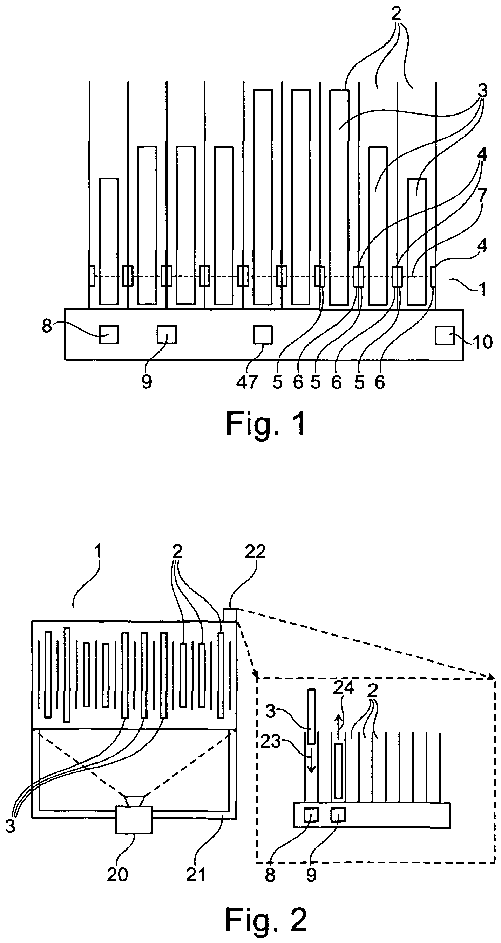

Die Fig. 1 zeigt eine Fachablage 1 umfassend mehrere Fächer 2 zum Ablegen von Röntgenbildplatten 3. Bei der dargestellten Ausführungsform ist das Registrierungssystem aus mehreren Sensoren 4 aufgebaut. Die Sensoren 4 sind Lichtschranken, die jeweils aus einer Lichtquelle 5 und einem optischen Sensor 6 aufgebaut sind. Beim Ablegen einer der Röntgenbildplatten 3 wird ein Lichtstrahl 7 zwischen der Lichtquelle 5 und dem optischen Sensor 6 unterbrochen. Der jeweilige optische Sensor 6 gibt dann ein Aktivierungssignal ab, das an eine Zeitmessvorrichtung 8 übermittelt wird. In der vorliegenden Ausführungsform ist die Zeitmessvorrichtung 8 in die Fachablage 1 integriert. Die Zeitvorrichtung könnte aber auch außerhalb der Fachlage angeordnet sein. Die Zeitmessvorrichtung 8 gibt dann auf das Aktivierungssignal hin einen Ablegezeitpunkt für jeden der Fächer 2 aus. Beim Entnehmen einer der Röntgenbildplatten 3 wird erneut ein Aktivierungssignal an die Zeitmessvorrichtung 8 übermittelt, so dass ein Entnahmezeitpunkt registriert wird. Darüber hinaus weist die Fachablage 1 einen Datenspeicher 9 auf, in dem die registrierten Ablegezeitpunkte und/oder Entnahmezeitpunkte sowie eine Fach-Identifikation des jeweiligen Fachs abgespeichert werden. Diese registrierten Informationen können ausgelesen werden, um eine eindeutige Identifizierung der Röntgenbildplatten zu ermöglichen. Die optischen Sensoren 6 sind mit einer Auswerteelektronik 47 versehen, die das Aktivierungssignal erzeugt. Die Fachablage 1 weist zusätzlich eine Sendeeinheit 10 auf, die die registrierten Informationen, wie die Fachablage-Identifikation, drahtlos, beispielsweise an eine Ausleseeinheit, übermittelt. Die Sendeeinheit könnte auch alternativ das Aktivierungssignal übermitteln.

Die Fig. 2 zeigt eine alternative Ausführungsform der Fachablage 1, wobei im Unterschied zu Fig. 1 das Registrierungssystem eine Kamera 20 aufweist, die mittels einer Halterung 21 positionsfest mit der Fachablage 1 verbunden ist. Die Kamera nimmt in kurzen zeitlichen Abständen mehrere Bilder von der Fachablage 1 auf, die mittels einer beispielsweise in der Fachablage integrierten elektronischen Analyseeinheit 22 ausgewertet werden. Die Analyseeinheit 22 kann dabei beispielsweise ein kompakter Mikrocomputer oder eine logische elektronische Schaltung, wie ein IC oder ein Prozessor, sein. Im gestrichelten Rahmen ist die Funktionsweise dieser Analyseeinheit 22 skizzenhaft dargestellt. Unter Verwendung eines Mustererkennungsverfahrens werden die einzelnen Fächer 2 in den aufgenommenen Bildern erkannt und der Belegungsstatus der einzelnen Fächer 2 überwacht. Beim Ablegen einer der Röntgenbildplatten, wie durch den Pfeil 23 dargestellt ist, wird dann ein Ablegezeitpunkt und beim Entnehmen eines der Röntgenbildplatten 3, wie durch den Pfeil 24 dargestellt ist, wird ein Entnahmezeitpunkt registriert. Auf diese Weise wird jedem der Fächer 2 der jeweilige Ablegezeitpunkt und/oder Entnahmezeitpunkt zugeordnet.

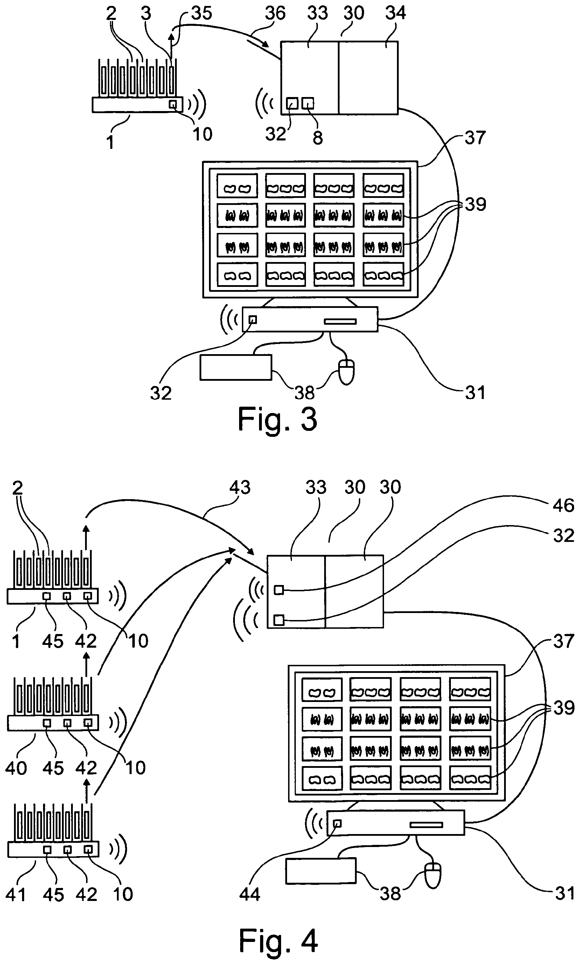

Die Fig. 3 zeigt zur Verdeutlichung des Registrierungsverfahrens die Fachablage 1, eine Auslesevorrichtung 30 und eine Bildarchivierungsvorrichtung 31. Die Fachablage 1 weist die Sendeeinheit 10 auf, die das Aktivierungssignal an eine Empfangseinheit 32 in der Auslesevorrichung 30 übermittelt. Die Empfangseinheit 32 kann ergänzend oder alternativ auch in die Bildarchivierungsvorrichtung 31 integriert sein. Die Zeitmessvorrichtung 8 ist in die Auslesevorrichtung 30 integriert. Falls die Röntgenbildplatten 3 Röntgenfilme sind, ist die Auslesevorrichtung 30 aus einer Filmentwicklungseinheit 33 für Röntgenfilme und einer Filmscaneinheit 34 aufgebaut. Die Röntgenbildplatten 3 werden, wie durch den Pfeil 35 dargestellt, in einer beliebigen Reihenfolge aus der Fachablage 1 entnommen und der Auslesevorrichtung 30, wie durch den Pfeil 36 dargestellt, zugeführt. Die Röntgenfilme 3 werden mittels der Filmentwicklungseinheit 33 entwickelt und anschließend mittels der Filmscaneinheit 34 gescannt, wobei digitale Aufnahmen des aufgenommenen Objekts erzeugt werden.

Falls die Röntgenbildplatten 3 Speicherfolien oder andere elektronische Röntgendetektoren sind, kann die Ausleseeinheit 30 entsprechend zum Auslesen dieser Röntgenbildplatten ausgestaltet sein und einen Speicherfolienscanner umfassen. Die Speicherfolien werden dem Speicherfolienscanner in der Ausleseeinheit 30 zugeführt, wobei digitale Aufnahmen des aufgenommenen Objekts erzeugt werden.

Die Bildarchivierungsvorrichtung 31, wie ein Computer, ist an eine Anzeigevorrichtung 37 und an Eingabemittel 38 angeschlossen. Die erzeugten digitalen Aufnahmen 39 werden von der Ausleseeinheit 30 an die Bildarchivierungsvorrichtung 31 übermittelt und mittels der Anzeigevorrichtung 37 dargestellt. Der Vorteil des vorliegenden Verfahrens liegt darin, dass die Röntgenbildplatten 3 in einer beliebigen Reihenfolge aus den Fächern 2 entnommen werden können, wobei der jeweilige Ablegezeitpunkt und/oder Entnahmezeitpunkt und die Fach-Identifikation des jeweiligen Fachs registriert werden. Dadurch ist die ursprüngliche Ablegereihenfolge der Röntgenbildplatten 3 bekannt, so dass die digitalen Aufnahmen 39 in der Bildarchivierungsvorrichtung automatisiert so umsortiert werden können, dass sie nach der ursprünglichen Ablagereihenfolge mittels der Anzeigevorrichtung 37 darstellbar sind. Dies erleichtert dem Benutzer die Beurteilung der digitalen Aufnahmen. Damit werden Verwechslungen der digitalen Aufnahmen, die Aufnahmegebieten zugeordnet sein können, vermieden.

Die Fig. 4 zeigt im Unterschied zur Fig. 3 mehrere Fachablagen, nämlich eine erste Fachablage 1, eine zweite Fachablage 40 und eine dritte Fachablage 41. In der ersten Fachablage 1 wurden Röntgenbildplatten 2 nach einer Aufnahmeserie eines ersten Patienten in der zweiten Fachablage 40 eines zweiten Patienten und in der dritten Fachablage 41 eines dritten Patienten abgelegt.

Zur Vermeidung von Patientenverwechselungen müssen die Fachablagen mit dem jeweils zu röntgenden Patienten gepaart werden. Dazu gibt es mehrere Alternativen. Bei einer ersten Alternative wird eine Paarungstaste verwendet, wobei beim betätigen die jeweilige Fachablage an den Patient gepaart wird.

Bei einer zweiten Alternative wird ein RFID-Chip verwendet, der bei der Annäherung an einen RFID-Reader ein Identifikationssignal abgibt und damit die Fachablage mit dem zu röntgenden Patienten gepaart wird.

Bei einer dritten Alternative wird ein visuell erkennbares Identifizierungskennzeichen an der Fachablage, wie ein farbiges oder ein alphanumerisches Zeichen, verwendet, welches mittels einer optischen Kamera erkannt wird und auf diese Weise die Fachablage mit dem zu röntgenden Patienten gepaart wird.

Bei der ersten Alternative mit der Paarungstaste wird vor Beginn der Ablage der Röntgenbildplatten 2 in die erste Fachablage 1 die Paarungstaste 42 an der Fachablage 1 durch den Benutzer betätigt, wobei mittels der Sendeeinheit 10 eine eindeutige Fachablage-Identifikation der ersten Fachablage 1 an die Empfangseinheit 32 der Auslesevorrichtung 30 übermittelt wird. Anschließend werden die Röntgenbildplatten 2 aus der Fachablage 1 entnommen und der Auslesevorrichtung 30 zugeführt, wie durch den Pfeil 43 dargestellt ist. Beim Entnehmen der Röntgenbildplatten 2 aus der Fachablage 1 werden die Registrierungsinformationen, wie z.B. die charakteristischen Eigenschaften der Röntgenbildplatte, der Ablegezeitpunkt, der Entnahmezeitpunkt, Fachablage-Identifikation und/oder die Fach-Identifikation, an die Empfangseinheit 32 übermittelt.

Auf diese Weise werden auch die zweite Fachablage 40 und die dritte Fachablage 41 gepaart und entladen. Dadurch können die digitalen Aufnahmen 39 dem ersten, zweiten oder dritten Patienten eindeutig zugeordnet werden. Zusätzlich kann vor dem Ablegen der Röntgenbildplatten in eine der Fachablagen 1, 40 oder 41 die jeweilige Paarungstaste 42 betätigt werden, so dass mittels der Bildarchivierungsvorrichtung 31 mehrere Fachablagen unterschiedlichen Patienten und Aufnahmeserien zugeordnet werden können. Die dargestellten digitalen Aufnahmen 39 sind dentale Aufnahmen eines Oberkiefers und eines Unterkiefers.

Alternativ oder ergänzend kann eine zweite Empfangseinheit 44 zum Empfang des Aktivierungssignals oder der Registrierungsdaten auch in die Bildarchivierungsvorrichtung 31 integriert sein. Dadurch kann eine herkömmliche Auslesevorrichtung ohne Empfangseinheit verwendet werden.

Bei der zweiten Alternative wird ein RFID-Chip 45 an jeder der Fachablagen 1, 40 und 41 verwendet, der bei der Annäherung an eine RFID-Empfangseinheit 46 ein Identifikationssignal abgibt und damit die Fachablage 1, 40 und 41 dem zu röntgenden Patienten zugeordnet wird.

### Bezugszeichen

- 1: Fachablage
- 2: Fächer
- 3: Röntgenbildplatten
- 4: Sensor; Lichtschranke
- 5: Lichtquelle
- 6: optischer Sensor
- 7: Lichtstrahl
- 8: Zeitmessvorrichtung
- 9: Datenspeicher
- 10: Sendeeinheit
- 20: Kamera
- 21: Halterung
- 22: Analyseeinheit
- 23: Ablegerichtung
- 24: Entnahmerichtung
- 30: Auslesevorrichtung/Speicherfolienscanner
- 31: Bildarchivierungsvorrichtung
- 32: Empfangseinheit
- 33: Filmentwicklungseinheit
- 34: Filmscaneinheit
- 35: Entnahmerichtung
- 36: Pfeil
- 37: Anzeigevorrichtung
- 38: Bedienelemente
- 39: Aufnahme
- 40: zweite Fachablage
- 41: dritte Fachablage
- 42: Paarungstaste
- 43: Pfeil für Transport der Röntgenbildplatte
- 44: zweite Empfangseinheit
- 45: RFID-Chip
- 46: RFID-Empfangseinheit
- 47: Auswerteelektronik

## Patentansprüche

1. Fachablage (1, 40, 41) umfassend mehrere Fächer (2) zum Ablegen von Röntgenbildplatten (3), **dadurch gekennzeichnet, dass** die Fachablage (1, 40, 41) ein Registrierungssystem (4, 20) aufweist, das beim Ablegen einer Röntgenbildplatte (3) in eines der Fächer (2) und/oder beim Entnehmen einer Röntgenbildplatte (3) aus einem der Fächer (2) aktiviert wird und dadurch ein Aktivierungssignal erzeugt, wobei eine Zeitmessvorrichtung (8) vorhanden ist, die auf das Aktivierungssignal hin einen Ablegezeitpunkt und/oder einen Entnahmezeitpunkt für die einzelnen Fächer (2) ausgibt, sobald das Registrierungssystem (4, 20) aktiviert wird, wobei beim Auslösen des Registrierungssystems der Ablegezeitpunkt und/oder der Entnahmezeitpunkt sowie eine Fach-Identifikation des jeweiligen Fachs und/oder einer Fachablage-Indikationen der jeweiligen Fachablage mittels eines Datenspeichers (9) registriert werden.

2. Fachablage (1, 40, 41) nach Anspruch 1, **dadurch gekennzeichnet, dass** das Registrierungssystem mindestens einen Sensor (4) für jedes der Fächer (2) aufweist, wobei die Fachablage eine Auswerteelektronik (47) aufweist, wobei beim Ablegen der Röntgenbildplatte (3) in eines der Fächer (2) und/oder beim Entnehmen der Röntgenbildplatte (3) aus einem der Fächer (2) der jeweilige Sensor (4) aktiviert wird und das Aktivierungssignal erzeugt wird.

3. Fachablage (1, 40, 41) nach Anspruch 1, **dadurch gekennzeichnet, dass** das Registrierungssystem eine Kamera (20) aufweist, die in kurzen zeitlichen Abständen mehrere Bilder von der Fachablage (1, 40, 41) aufnimmt, wobei mittels einer Analyseeinheit (22) durch computergestützten Vergleich einzelner Bilder oder Bildsequenzen beim Ablegen und/oder beim Entnehmen der Röntgenbildplatte (3) der Ablegezeitpunkt und/oder der Entnahmezeitpunkt sowie die Fach-Identifikation des jeweiligen Fachs ermittelt werden.

4. Fachablage (1, 40, 41) nach Anspruch 3, **dadurch gekennzeichnet, dass** die Bilder oder Bildsequenzen unter Verwendung eines Mustererkennungsverfahrens analysiert werden, um den Belegungsstatus der einzelnen Fächer (2) festzustellen.

5. Fachablage (1, 40, 41) nach einem der Ansprüche 1 bis 4, **dadurch gekennzeichnet, dass** die Fachablage (1, 40, 41) eine Identifikationselektronik aufweist, die beim Ablegen und/oder beim Entnehmen der Röntgenbildplatte (3) die Fach-Identifikation des jeweiligen Fachs ausgibt.

6. Fachablage (1, 40, 41) nach einem der Ansprüche 1 bis 5, **dadurch gekennzeichnet, dass** die Röntgenbildplatten (3) Speicherfolien, Röntgenfilme oder elektronische Röntgenbilddetektoren sind.

7. Fachablage (1, 40, 41) nach einem der Ansprüche 1 bis 6, **dadurch gekennzeichnet, dass** die Fachablage (1, 40, 41) unter Verwendung von Befestigungsmitteln an eine Auslesevorrichtung (30) anbringbar oder in die Auslesevorrichtung (30) integriert ist.

8. Fachablage (1, 40, 41) nach einem der Ansprüche 1 bis 7, **dadurch gekennzeichnet, dass** die Zeitmessvorrichtung (8) in der Fachablage (1, 40, 41), in eine Bildarchivierungsvorrichtung (31) oder in die Auslesevorrichtung (30) integriert ist.

9. Fachablage (1, 40, 41) nach einem der Ansprüche 1 bis 8, **dadurch gekennzeichnet, dass** der Datenspeicher (9) in die Fachablage (1, 40, 41), in die Auslesevorrichtung (30) oder in eine Bildarchivierungsvorrichtung integriert ist.

10. Fachablage (1, 40, 41) nach einem der Ansprüche 1 bis 9, **dadurch gekennzeichnet, dass** die Analyseeinheit (22) in die Fachablage (1, 40, 41), in die Auslesevorrichtung (30) oder in die Bildarchivierungsvorrichtung integriert ist.

11. Fachablage (1, 40, 41) nach einem der Ansprüche 1 bis 10, **dadurch gekennzeichnet, dass** die Sensoren (4) charakteristische Eigenschaften der Röntgenbildplatte (3) detektieren, wobei diese Information zusammen mit dem Ablegezeitpunkt und/oder dem Entnahmezeitpunkt mittels des Datenspeichers (9) registrierbar sind.

12. Fachablage (1, 40, 41) nach einem der Ansprüche 1 bis 11, **dadurch gekennzeichnet, dass** die Fachablage (1, 40, 41) eine Paarungstaste (42) aufweist.

13. Fachablage (1, 40, 41) nach einem der Ansprüche 1 bis 11, **dadurch gekennzeichnet, dass** die Fachablage einen individuellen RFID-Chip (46) zur Identifikation aufweist.

14. Fachablage (1, 40, 41) nach einem der Ansprüche 1 bis 11, **dadurch gekennzeichnet, dass** die Fachablage ein visuell erkennbares Identifizierungskennzeichen aufweist.

15. Verfahren zur Registrierung von Röntgenbildplatten (3), wobei mehrere Röntgenbildplatten (3) in einer Fachablage (1, 40, 41) abgelegt werden, wobei die Fachablage (1, 40, 41) mehrere Fächer (2) aufweist, **dadurch gekennzeichnet, dass** die Fachablage (1, 40, 41) ein Registrierungssystem aufweist, das beim Ablegen einer Röntgenbildplatte (3) in eines der Fächer (2) und/oder beim Entnehmen einer Röntgenbildplatte (3) aus einem der Fächer (2) aktiviert wird und dadurch ein Aktivierungssignal erzeugt wird, wobei eine Zeitmessvorrichtung (8) vorhanden ist, die auf das Aktivierungssignal hin einen Ablegezeitpunkt und/oder einen Entnahmezeitpunkt für die einzelnen Fächer (2) ausgibt, sobald das Registrierungssystem aktiviert wird, wobei beim Auslösen des Registrierungssystems der Ablegezeitpunkt, der Entnahmezeitpunkt, eine Fach-Identifikation des jeweiligen Fachs und/oder eine Fachablage-Identifikation der jeweiligen Fachablage mittels eines Datenspeichers (9) registriert werden.

16. Verfahren nach Anspruch 15, **dadurch gekennzeichnet, dass** das Registrierungssystem jeweils mindestens einen Sensor (4) für jedes der Fächer (2) aufweist, die jeweils mit einer Auswerteelektronik (47) versehen sind, wobei beim Ablegen der Röntgenbildplatte (3) in eines der Fächer (2) und/oder beim Entnehmen der Röntgenbildplatte (3) aus einem der Fächer (2) der jeweilige Sensor (4) aktiviert wird und das Aktivierungssignal erzeugt wird.

17. Verfahren nach Anspruch 15, **dadurch gekennzeichnet, dass** das Registrierungssystem eine Kamera (20) aufweist, die zur Überwachung in kurzen zeitlichen Abständen mehrere Bilder der Fachablage (1, 40, 41) aufnimmt, wobei durch computergestützten Vergleich einzelner Bilder oder Bildsequenzen auf charakteristische Strukturen hin beim Ablegen und/oder beim Entnehmen der Röntgenbildplatte (3) der Ablegezeitpunkt und/oder der Entnahmezeitpunkt sowie die Fach-Identifikation des jeweiligen Fachs ermittelt werden.

18. Verfahren nach Anspruch 17, **dadurch gekennzeichnet, dass** die Bilder unter Verwendung eines Mustererkennungsverfahrens analysiert werden, um den Belegungsstatus der einzelnen Fächer (2) festzustellen.

19. Verfahren nach einem der Ansprüche 15 bis 18, **dadurch gekennzeichnet, dass** die Röntgenbildplatten (3) aus der Fachablage (1, 40, 41) herausgenommen und mittels einer Auslesevorrichtung (30) die Röntgenbildplatten (3) ausgelesen werden, wobei digitale Aufnahmen (39) erzeugt werden und an eine Bildarchivierungsvorrichtung (31) übermittelt werden.

20. Verfahren nach Anspruch 19, **dadurch gekennzeichnet, dass** unter Verwendung der Zuordnung zwischen dem registrierten Ablegezeitpunkt und der registrierten Fach-Identifikation für jeden der Fächer (2) die erzeugten digitalen Aufnahmen (39) mittels der Bildarchivierungsvorrichtung (31) und/oder der Auslesevorrichtung (30) so umsortiert werden, dass sie nach einer ursprünglichen Ablagereihenfolge angeordnet sind.

21. Verfahren nach Anspruch 19 oder 20, **dadurch gekennzeichnet, dass** ein Fachablagesystem mehrere Fachablagen (1, 40, 41) aufweist, wobei jede der Fachablagen (1, 40, 41) eine Paarungstaste (42) aufweist, wobei die Paarungstaste (42) zur Paarung von Fachablage und eines zu röntgenden Patienten als Bestätigung betätigt wird, wobei dabei dem Anwender die Identität der Fachablage und ein Patientenname des zu röntgenden Patienten mittels einer Anzeigeeinheit der Bildarchivierungseinrichtung (31) oder der Auslesevorrichtung (30) angezeigt werden, wobei die Paarung unter Verwendung von Bedienelemente (38) an der Bildarchivierungseinrichtung (31) oder an der Auslesevorrichtung (30) durch den Benutzer manuell bestätigt wird.

22. Verfahren nach Anspruch 19 oder 20, **dadurch gekennzeichnet, dass** ein Fachablagesystem mehrere Fachablagen (1, 40, 41) aufweist, wobei jede der Fachablagen (1, 40, 41) einen individuellen RFID-Chip (46) zur Identifikation aufweist, wobei die jeweilige Fachablage (1, 40, 41) zur Paarung von Fachablage und eines zu röntgenden Patienten in einen Empfangsradius einer RFID-Empfangseinheit (47) gebracht wird, wobei daraufhin der jeweilige RFID-Chip ein eindeutiges Identifikationssignal an die RFID-Empfangseinheit übermittelt, wobei dabei dem Anwender die Identität der Fachablage und ein Patientenname des zu röntgenden Patienten mittels einer Anzeigeeinheit der Bildarchivierungseinrichtung (31) oder der Auslesevorrichtung (30) angezeigt werden, wobei die Paarung unter Verwendung von Bedienelemente (38) an der Bildarchivierungseinrichtung (31) oder an der Auslesevorrichtung (30) durch den Benutzer manuell bestätigt wird.

23. Verfahren nach Anspruch 19 oder 20, **dadurch gekennzeichnet, dass** ein Fachablagesystem mehrere Fachablagen (1, 40, 41) aufweist, wobei sich die Fachablagen (1, 40, 41) durch visuell erkennbare Identifizierungskennzeichen unterscheiden, wobei die jeweilige Fachablage (1, 40, 41) zur Paarung von Fachablage und eines zu röntgenden Patienten anhand der Identifizierungskennzeichen identifiziert werden, wobei dabei dem Anwender die Identität der Fachablage und ein Patientenname des zu röntgenden Patienten mittels einer Anzeigeeinheit der Bildarchivierungseinrichtung (31) oder der Auslesevorrichtung (30) angezeigt werden, wobei die Paarung unter Verwendung von Bedienelemente (38) an der Bildarchivierungseinrichtung (31) oder an der Auslesevorrichtung (30) durch den Benutzer manuell bestätigt wird.

## Claims

1. Tray repository (1, 40, 41) comprising multiple trays (2) for depositing x-ray image plates (3), **characterized in that** the tray repository (1, 40, 41) has a registration system (4, 20) that is activated upon depositing an x-ray image plate (3) in one of the trays (2) and/or upon removal of an x-ray image plate (3) from one of the trays (2) and thereby generates an activation signal, wherein a time measurement device (8) is present that, in response to the activation signal, outputs a deposition time point and/or a removal time point for the individual trays (2) as soon as the registration system (4, 20) is activated, wherein, upon triggering of the registration system, the deposition time point and/or the removal time point, as well as a tray identification of the respective tray and/or of a tray repository indications of the respective tray repository, are registered by means of a data storage unit (9).

2. Tray repository (1, 40, 41) according to claim 1, **characterized in that** the registration system has at least one sensor (4) for each of the trays (2), wherein the tray repository has an electronic evaluation unit (47), wherein the respective sensor (4) is activated and the activation signal is generated upon depositing the x-ray image plate (3) in one of the trays (2) and/or upon removal of the x-ray image plate (3) from one of the trays (2).

3. Tray repository (1, 40, 41) according to claim 1, **characterized in that** the registration system has a camera (20) that takes multiple images of the tray repository (1, 40, 41) at short time intervals, wherein, upon deposition and/or removal of the x-ray image plate (3), the deposition time point and/or the removal time point, as well as the tray identification of the respective tray, are determined by means of an analysis unit (22) via computer-aided comparison of individual images or image sequences.

4. Tray repository (1, 40, 41) according to claim 3, **characterized in that** the images or image sequences are analyzed using a pattern recognition method in order to establish the occupation status of the individual trays (2).

5. Tray repository (1, 40, 41) according to one of claims 1 through 4, **characterized in that** the tray repository (1, 40, 41) has an electronic identification unit that outputs the tray identification of the respective tray upon deposition and/or removal of the x-ray image plate (3).

6. Tray repository (1, 40, 41) according to one of claims 1 through 5, **characterized in that** the x-ray image plates (3) are imaging plates, x-ray films, or electronic x-ray image detectors.

7. Tray repository (1, 40, 41) according to one of claims 1 through 6, **characterized in that** the tray repository (1, 40, 41) can be attached to a readout device (30) using attachment means, or is integrated into the readout device (30).

8. Tray repository (1, 40, 41) according to one of claims 1 through 7, **characterized in that** the time measurement device (8) is integrated into the tray repository (1, 40, 41), into an image archiving device (31), or into the readout device (30).

9. Tray repository (1, 40, 41) according to one of claims 1 through 8, **characterized in that** the data storage unit (9) is integrated into the tray repository (1, 40, 41), into the readout device (30), or into an image archiving device.

10. Tray repository (1, 40, 41) according to one of claims 1 through 9, **characterized in that** the analysis unit (22) is integrated into the tray repository (1, 40, 41), into the readout device (30), or into the image archiving device.

11. Tray repository (1, 40, 41) according to one of claims 1 through 10, **characterized in that** the sensors (4) detect characteristic properties of the x-ray image plate (3), wherein this information, together with the deposition time point and/or the removal time point, can be registered by means of the data storage unit (9).

12. Tray repository (1, 40, 41) according to one of claims 1 through 11, **characterized in that** the tray repository (1, 40, 41) has a pairing button (42).

13. Tray repository (1, 40, 41) according to one of claims 1 through 11, **characterized in that** the tray repository has an individual RFID chip (46) for identification.

14. Tray repository (1, 40, 41) according to one of claims 1 through 11, **characterized in that** the tray repository has a visually recognizable identification marking.

15. Method for registration of x-ray image plates (3), wherein multiple x-ray image plates (3) are stored in a tray repository (1, 40, 41), wherein the tray repository (1, 40, 41) has multiple trays (2), **characterized in that** the tray repository (1, 40, 41) has a registration system that is activated upon depositing an x-ray image plate (3) in one of the trays (2) and/or upon removal of an x-ray image plate (3) from one of the trays (2) and an activation signal is thereby generated, wherein a time measurement device (8) is present that, in response to the activation signal, outputs a deposition time point and/or a removal time point for the individual trays (2) as soon as the registration system is activated, wherein, upon triggering of the registration system, the deposition time point, the removal time point, a tray identification of the respective tray, and/or a tray repository identification of the respective tray repository are registered by means of a data storage unit (9).

16. Method according to claim 15, **characterized in that** the registration system has at least one sensor (4) respectively for each of the trays (2), which are respectively provided with an electronic evaluation unit (47), wherein the respective sensor (4) is activated and the activation signal is generated upon depositing the x-ray image plate (3) in one of the trays (2) and/or upon removal of the x-ray image plate (3) from one of the trays (2).

17. Method according to claim 15, **characterized in that** the registration system has a camera (20) that, for monitoring, takes multiple images of the tray repository (1, 40, 41) at short time intervals, wherein, upon deposition and/or removal of the x-ray image plate (3), the deposition time point and/or the removal time point, as well as the tray identification of the respective tray, are determined via computer-aided comparison of individual images or image sequences for characteristic structures.

18. Method according to claim 17, **characterized in that** the images are analyzed using a pattern recognition method in order to establish the occupation status of the individual trays (2).

19. Method according to one of claims 15 through 18, **characterized in that** the x-ray image plates (3) are removed from the tray repository (1, 40, 41) and read out by means of a readout device (30), wherein digital exposures (39) are generated and transmitted to an image archiving device (31).

20. Method according to claim 19, **characterized in that** the generated digital exposures (39) are re-sorted by means of the image archiving device (31) and/or the readout device (30), using the association between the registered deposition time point and the registered tray identification for each of the trays (2), so that they are arranged according to an original deposition sequence.

21. Method according to claim 19 or 20, **characterized in that** a tray repository system has multiple tray repositories (1, 40, 41), wherein each of the tray repositories (1, 40, 41) has a pairing button (42); wherein the pairing button (42) is actuated, as confirmation, for pairing tray repository and a patient to be x-rayed; wherein the identity of the tray repository and a patient name of the patient to be x-rayed are thereby indicated to the user by means of a display unit of the image archiving device (31) or of the readout device (30); wherein the pairing is confirmed manually by the user using control elements (38) at the image archiving device (31) or of the readout device (30).

22. Method according to claim 19 or 20, **characterized in that** a tray repository system has multiple tray repositories (1, 40, 41), wherein each of the tray repositories (1, 40, 41) has an individual RFID chip (46) for identification; wherein the respective tray repository (1, 40, 41) is brought within a reception radius of an RFID receiver unit (47) for pairing tray repository and a patient to be x-rayed; wherein the respective RFID chip thereupon transmits a unique identification signal to the RFID receiver unit; wherein the identity of the tray repository and a patient name of the patient to be x-rayed are thereby indicated to the user by means of a display unit of the image archiving device (31) or of the readout device (30); wherein the pairing is confirmed manually by the user using control elements (38) at the image archiving device (31) or of the readout device (30).

23. Method according to claim 19 or 20, **characterized in that** a tray repository system has multiple tray repositories (1, 40, 41), wherein the tray repositories (1, 40, 41) differ via visually recognizable identification markings; wherein the respective tray repository (1, 40, 41) are identified using the identification markings for pairing tray repository and a patient to be x-rayed; wherein the identity of the tray repository and a patient name of the patient to be x-rayed are thereby indicated to the user by means of a display unit of the image archiving device (31) or of the readout device (30); wherein the pairing is confirmed manually by the user using control elements (38) at the image archiving device (31) or of the readout device (30).

## Revendications

1. Bac compartimenté (1, 40, 41) comprenant plusieurs compartiments (2) servant à ranger des plaques radiographiques (3), **caractérisé en ce que** le bac compartimenté (1, 40, 41) présente un système d'enregistrement (4, 20), qui peut être activé lorsqu'une plaque radiographique (3) est rangée dans l'un des compartiments (2) et/ou lorsqu'une plaque radiographique (3) est retirée de l'un des compartiments (2) et qui génère ainsi un signal d'activation, dans lequel un dispositif de mesure de temps (8) est disponible, qui délivre en réponse au signal d'activation un moment de rangement et/ou un moment de retrait pour chaque compartiment (2), dès que le système d'enregistrement (4, 20) est activé, dans lequel, lors du déclenchement du système d'enregistrement, le moment de rangement et/ou le moment de retrait, ainsi qu'une identification du compartiment correspondant et/ou une identifications du bac compartimenté correspondant sont enregistrés au moyen d'une mémoire de données (9).

2. Bac compartimenté (1, 40, 41) selon la revendication 1, **caractérisé en ce que** le système d'enregistrement présente au moins un capteur (4) pour chacun des compartiments (2), dans lequel le bac compartimenté présente un dispositif électronique d'évaluation (47), dans lequel lors du rangement de la plaque radiologique (3) dans un des compartiments (2) et/ou lors du retrait de la plaque radiologique (3) depuis un des compartiments (2), le capteur correspondant (4) est activé et le signal d'activation est généré.

3. Bac compartimenté (1, 40, 41) selon la revendication 1, **caractérisé en ce que** le système d'enregistrement présente une caméra (20), qui capture selon une périodicité rapprochée plusieurs images du bac compartimenté (1, 40, 41), dans lequel, au moyen d'une unité d'analyse (22), par comparaison assistée par ordinateur des séquences d'images ou images individuelles, le moment de rangement et/ou le moment de retrait sont déterminés lors du rangement et/ou lors du retrait de la plaque radiographique (3) ainsi que l'identification du compartiment correspondant.

4. Bac compartimenté (1, 40, 41) selon la revendication 3, **caractérisé en ce que** les images ou séquences d'images sont analysées par utilisation d'un procédé de reconnaissance des formes, afin de déterminer l'état d'occupation des compartiments individuels (2).

5. Bac compartimenté (1, 40, 41) selon l'une des revendications 1 à 4, **caractérisé en ce que** le bac compartimenté (1, 40, 41) présente un dispositif électronique d'identification, qui délivre l'identification du compartiment individuel lors du rangement et/ou du retrait de la plaque radiographique (3).

6. Bac compartimenté (1, 40, 41) selon l'une des revendications 1 à 5, **caractérisé en ce que** les plaques radiographiques (3) sont des films-mémoires, des films radiographiques ou des détecteurs d'images radiographiques électroniques.

7. Bac compartimenté (1, 40, 41) selon l'une des revendications 1 à 6, **caractérisé en ce que** le bac compartimenté (1, 40, 41) est monté sur un dispositif de lecture (30) à l'aide d'un moyen de fixation ou intégré dans le dispositif de lecture (30).

8. Bac compartimenté (1, 40, 41) selon l'une des revendications 1 à 7, **caractérisé en ce que** le dispositif de mesure de temps (8) est intégré dans le bac compartimenté (1, 40, 41), dans un dispositif d'archivage d'image (31) ou dans le dispositif de lecture (30).

9. Bac compartimenté (1, 40, 41) selon l'une des revendications 1 à 8, **caractérisé en ce que** la mémoire de données (9) est intégrée dans le bac compartimenté (1, 40, 41), dans le dispositif de lecture (30) ou dans un dispositif d'archivage d'image.

10. Bac compartimenté (1, 40, 41) selon l'une des revendications 1 à 9, **caractérisé en ce que** l'unité d'analyse (22) est intégrée dans le bac compartimenté (1, 40, 41), dans le dispositif de lecture (30) ou dans le dispositif d'archivage d'image.

11. Bac compartimenté (1, 40, 41) selon l'une des revendications 1 à 10, **caractérisé en ce que** les capteurs (4) détectent des propriétés caractéristiques de la plaque radiographique (3), dans lequel ces informations peuvent être enregistrées conjointement avec le moment de rangement et/ou le moment de retrait au moyen de la mémoire de données (9).

12. Bac compartimenté (1, 40, 41) selon l'une des revendications 1 à 11, **caractérisé en ce que** le bac compartimenté (1, 40, 41) présente une touche d'appariement (42).

13. Bac compartimenté (1, 40, 41) selon l'une des revendications 1 à 11, **caractérisé en ce que** le bac compartimenté présente une puce RFID individuelle (46) pour l'identification.

14. Bac compartimenté (1, 40, 41) selon l'une des revendications 1 à 11, **caractérisé en ce que** le bac compartimenté présente une marque d'identification visuellement reconnaissable.

15. Procédé d'enregistrement de plaques radiographiques (3), dans lequel plusieurs plaques radiographiques (3) sont rangées dans un bac compartimenté (1, 40, 41), dans lequel le bac compartimenté (1, 40, 41) présente plusieurs compartiments (2), **caractérisé en ce que** le bac compartimenté (1, 40, 41) présente un système d'enregistrement, qui est activé lorsqu'une plaque radiographique (3) est rangée dans l'un des compartiments (2) et/ou lorsqu'une plaque radiographique (3) est retirée de l'un des compartiments (2) et qui génère ainsi un signal d'activation, dans lequel un dispositif de mesure de temps (8) est disponible, qui délivre en réponse au signal d'activation un moment de rangement et/ou un moment de retrait pour chaque compartiment (2), dès que le système d'enregistrement est activé, dans lequel, lors du déclenchement du système d'enregistrement, le moment de rangement, le moment de retrait, une identification du compartiment correspondant et/ou une identification du bac compartimenté correspondant sont enregistrés au moyen d'une mémoire de données (9).

16. Procédé selon la revendication 15, **caractérisé en ce que** le système d'enregistrement présente au moins un capteur (4) pour chacun des compartiments (2), qui présente chacun un dispositif électronique d'évaluation (47), dans lequel lors du rangement de la plaque radiologique (3) dans un des compartiments (2) et/ou lors du retrait de la plaque radiologique (3) depuis un des compartiments (2), le capteur correspondant (4) est activé et le signal d'activation est généré.

17. Procédé selon la revendication 15, **caractérisé en ce que** le système d'enregistrement présente une caméra (20), qui capture à des fins de surveillance selon une périodicité rapprochée plusieurs images du bac compartimenté (1, 40, 41), dans lequel, par comparaison assistée par ordinateur des séquences d'images ou images individuelles à des structures caractéristiques, le moment de rangement et/ou le moment de retrait sont déterminés lors du rangement et/ou lors du retrait de la plaque radiographique (3) ainsi que l'identification du compartiment correspondant.

18. Procédé selon la revendication 17, **caractérisé en ce que** les images sont analysées par utilisation d'un procédé de reconnaissance des formes, afin de déterminer l'état d'occupation des compartiments individuels (2).

19. Procédé selon l'une des revendications 15 à 18, **caractérisé en ce que** les plaques radiographiques (3) sont retirées du bac compartimenté (1, 40, 41) et les plaques radiographiques (3) sont lues au moyen d'un dispositif de lecture (30), dans lequel des prises de vue numériques (39) sont produites et transmises à un dispositif d'archivage d'image (31).

20. Procédé selon la revendication 19, **caractérisé en ce que**, en utilisant le rattachement entre le moment de rangement enregistré et l'identification de compartiment enregistrée pour chacun des compartiments (2), les prises de vue numériques (39) produites sont triées au moyen du dispositif d'archivage d'image (31) et/ou du dispositif de lecture (30) de sorte qu'elles soient disposées selon un ordre de rangement initial.

21. Procédé selon la revendication 19 ou 20, **caractérisé en ce qu'**un système de bacs compartimentés présente plusieurs bacs compartimentés (1, 40, 41), dans lequel chacun des bacs compartimentés (1, 40, 41) présente une touche d'appariement (42), dans lequel la touche d'appariement (42) pour l'appariement de bacs compartimentés et d'un patient à radiographier est actionnée sous la forme d'une confirmation, dans lequel l'identité du bac compartimenté et un nom du patient à radiographier sont alors affichés pour l'utilisateur au moyen d'une unité d'affichage du dispositif d'archivage d'image (31) ou du dispositif de lecture (30), dans lequel l'appariement est actionné manuellement par l'utilisateur en utilisant des éléments de commande (38) sur le dispositif d'archivage d'image (31) ou sur le dispositif de lecture (30).

22. Procédé selon la revendication 19 ou 20, **caractérisé en ce qu'**un système de bacs compartimentés présente plusieurs bacs compartimentés (1, 40, 41), dans lequel chacun des bacs compartimentés (1, 40, 41) présente une puce RFID individuelle (46) pour l'identification, dans lequel le bac compartimenté (1, 40, 41) correspondant est amené dans un rayon de réception d'une unité de réception RFID (47) pour l'appariement du bac compartimenté avec un patient à radiographier, dans lequel la puce RFID correspondante transmis alors un signal d'identification unique à l'unité de réception RFID, dans lequel l'identité du bac compartimenté et un nom du patient à radiographier sont alors affichés pour l'utilisateur au moyen d'une unité d'affichage du dispositif d'archivage d'image (31) ou du dispositif de lecture (30), dans lequel l'appariement est actionné manuellement par l'utilisateur en utilisant des éléments de commande (38) sur le dispositif d'archivage d'image (31) ou sur le dispositif de lecture (30).

23. Procédé selon la revendication 19 ou 20, **caractérisé en ce qu'**un système de bacs compartimentés présente plusieurs bacs compartimentés (1, 40, 41), dans lequel les bacs compartimentés (1, 40, 41) se différencient par des marques d'identification visuellement reconnaissables, dans lequel le bac compartimenté (1, 40, 41) respectifs sont identifiés pour l'appariement de bacs compartimentés et d'un patient à radiographier à l'aide des marques d'identification, dans lequel l'identité du bac compartimenté et un nom du patient à radiographier sont alors affichés pour l'utilisateur au moyen d'une unité d'affichage du dispositif d'archivage d'image (31) ou du dispositif de lecture (30), dans lequel l'appariement est actionné manuellement par l'utilisateur en utilisant des éléments de commande (38) sur le dispositif d'archivage d'image (31) ou sur le dispositif de lecture (30).
